# EUROPEAN PATENT APPLICATION

(11) **EP 4 782 007 A1**
(43) Date of publication of application: **29.07.2026**
(21) Application number: 24868251.0
(22) Date of filing: 17.09.2024
(51) Int. Cl.: A61K 47/32, A61K 9/08, A61K 47/18, A61K 47/38, A61P 27/02, A61P 43/00

(54) **OPHTHALMIC COMPOSITION**

(30) Priority: 19.09.2023 JP 2023150869
(71) Applicant: NOF Corporation, Shibuya-ku Tokyo 150-6019 (JP)
(72) Inventor: SUMI, Azusa, Kawasaki-shi, Kanagawa 210-0865 (JP); IWAKIRI, Norio, Kawasaki-shi, Kanagawa 210-0865 (JP)
(74) Representative: Müller-Boré & Partner Patentanwälte PartG mbB
(86) International application number: PCT/JP2024/033128
(87) International publication number: WO 2025/063174

(57) **Abstract**

An ophthalmic composition according to the present invention includes (A) 0.01 w/w% or more and 0.5 w/w% or less of a copolymer having a constitutional unit represented by the following formula (1a) to formula (1c), a molar ratio of each constitutional unit of a: b: c = 100: 10 to 400: 2 to 50, and a weight average molecular weight of 5,000 to 2,000,000, and (B) 0.001 w/w% or more and 0.02 w/w% or less of benzalkonium chloride, and/or (C) 0.001 w/w% or more and 0.05 w/w% or less of chlorhexidine gluconate. According to the present invention, an ophthalmic composition that exhibits sufficient preservative efficacy and protects corneal cells can be provided.

## Description

### Technical Field

The present invention relates to an ophthalmic composition.

### Background Art

In recent years, due to an increase in the number of contact lens wearers and the spread of digital devices, the number of dry eye patients who complain of discomfort and dryness of eyes has increased. In addition to dry eye symptoms, many people have recently complained of pollen allergies, the number of patients with pollen allergies has been rapidly increasing in the last 10 years, and the number of people who use ophthalmic solutions for measures and treatment of these symptoms has been increasing year by year.

Generally, since ophthalmic solutions are repeatedly used for several weeks after opening, a preservative is blended in order to prevent bacterial contamination in the container. While the preservative contributes to the preservative efficacy of the ophthalmic solution, when the ophthalmic solution is used over a long period of time without following the usage and dosage, there is a possibility of causing superficial punctate keratopathy caused by death of some cells on the corneal surface.

In addition, there are many patients with pollen allergies who use ophthalmic solutions exceeding a predetermined number of time for ocular instillation in a short time for the purpose of washing off pollen. In this case, there is a possibility of causing side effects such as corneal disorder and blepharitis, by the preservative. For these reasons, single-use type ophthalmic solutions containing no preservative have been developed in recent years, and there is a demand for an ophthalmic solution that is gentler on the eyes.

In currently commercially available ophthalmic solutions, benzalkonium chloride, chlorhexidine gluconate, or the like is used as a preservative. Particularly, benzalkonium chloride is used in many ophthalmic solutions because it has a wide antibacterial range. In this regard, there is a possibility that ophthalmic solutions using these preservatives induce a corneal epithelial disorder when the use frequency or concentration is high, even though the ophthalmic solutions are not instilled for a long period of time.

Generally, when the secretion amount of tear is maintained at a normal level, the concentration of the preservative decreases due to the turnover of tear, and therefore it does not affect the surface of the eye. However, when the secretion amount of tear is reduced due to a dry eye symptom, the preservative remains on the surface of the eye for a long time, and there is a possibility that it affects the surface of the eye even at a low concentration. Further, when the cornea is damaged or in the case of a patient requiring frequent ocular instillation, there is a possibility that corneal disorder or corneal infection is caused. Therefore, there is a demand for an ophthalmic solution that exhibits sufficient preservative efficacy and protects corneal cells from preservatives so that it can be safely used by all patients.

In recent years, additives for protecting cells from cytotoxic substances have been developed. For example, NPL 1 reports a method for protecting cells from benzalkonium chloride as a preservative by blending D-mannitol in an ophthalmic solution.

Further, PTL 1 discloses an ophthalmic solution containing a phosphorylcholine group-containing polymer and polyhexamethylene biguanide. By using the ophthalmic solution described in PTL 1, it has sufficient preservative efficacy, quickly spreads on the surface of an eye or a contact lens, and exhibits moisturizing and lubricating effects.

### Citation List

### Patent Literature

PTL 1: JP2021-20856A

### Non-Patent Literature

NPL 1: Journal of Oleo Science, Noriaki Nagai, 2015, vol. 64, no. 7

### Summary of Invention

### Technical Problem

Although NPL 1 is a method for protecting cells from a preservative of an ophthalmic solution, it does not clearly describe a phosphorylcholine group-containing polymer. In addition, when a sufficient amount of the specific ternary copolymer that greatly contributes to the improvement of lubricity described in PTL 1 is blended, there is a case where sufficient preservative efficacy is not obtained depending on the type of the preservative.

In view of the above problems, an object of the present invention is to provide an ophthalmic composition that exhibits sufficient preservative efficacy and protects corneal cells.

### Solution to Problem

As a result of intensive studies, the present inventors have found that an ophthalmic composition containing specific amounts of a copolymer having a constitutional unit represented by formula (1a) to formula (1c), a molar ratio of each constitutional unit of a: b: c = 100: 10 to 400: 2 to 50, and a weight average molecular weight of 5,000 to 2,000,000 (hereinafter, also referred to as a "copolymer (P)") and a specific preservative can exhibit sufficient preservative efficacy and protect corneal cells, thereby completing the present invention.

That is, the present invention is as follows.
[1] An ophthalmic composition containing (A) 0.01 w/w% or more and 0.5 w/w% or less of a copolymer having a constitutional unit represented by the following formula (1a) to formula (1c), a molar ratio of each constitutional unit of a: b: c = 100: 10 to 400: 2 to 50, and a weight average molecular weight of 5,000 to 2,000,000, and (B) 0.001 w/w% or more and 0.02 w/w% or less of benzalkonium chloride, and/or (C) 0.001 w/w% or more and 0.05 w/w% or less of chlorhexidine gluconate. 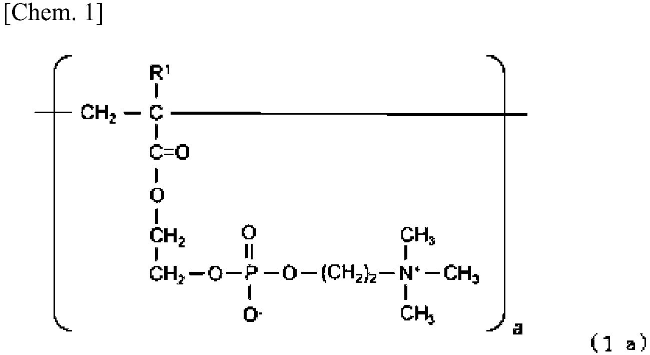 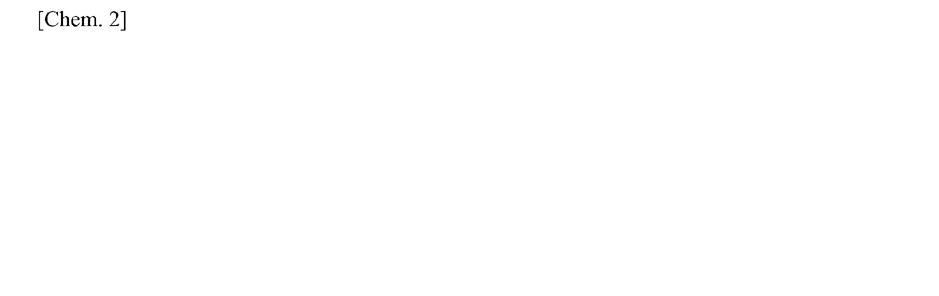 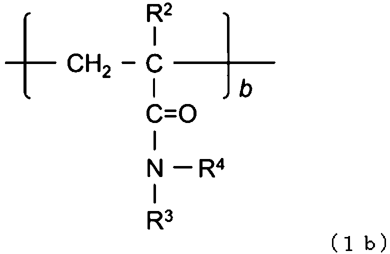 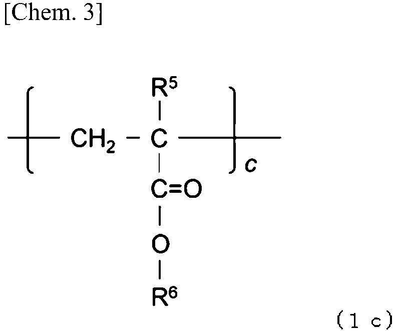 [in the formula (1a) to formula (1c), a, b, and c each represent a molar ratio of each constitutional unit; R¹, R² and R⁵ each independently represent a hydrogen atom or a methyl group; R³ and R⁴ each independently represent a hydrogen atom, a methyl group or an ethyl group; and R⁶ represents a monovalent hydrocarbon group having 12 to 24 carbon atoms.]
[2] The ophthalmic composition according to [1], containing 0.5 w/w% or less of hydroxypropyl methylcellulose.

### Advantageous Effects of Invention

The ophthalmic composition of the present invention can protect corneal cells while exhibiting sufficient preservative efficacy. That is, the present invention can provide an ophthalmic composition excellent in protection of corneal cells.

### Description of Embodiments

The ophthalmic composition according to the present invention includes 0.01 w/w% or more and 0.5 w/w% or less of a copolymer having a constitutional unit represented by the following formula (1a) to formula (1c), a molar ratio of each constitutional unit of a: b: c = 100: 10 to 400: 2 to 50, and a weight average molecular weight of 5,000 to 2,000,000, as a component (A); and 0.001 w/w% or more and 0.02 w/w% or less of benzalkonium chloride, as a component (B); and/or 0.001 w/w% or more and 0.05 w/w% or less of chlorhexidine gluconate, as a component (C).

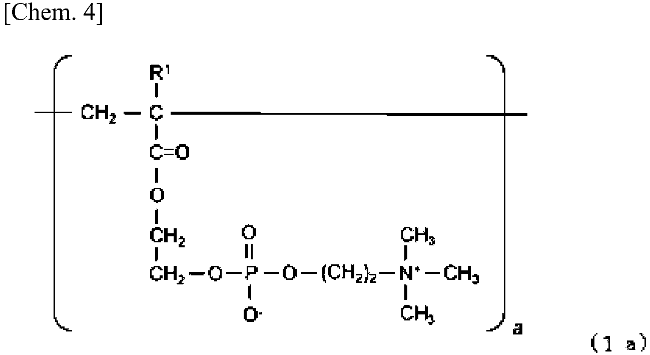

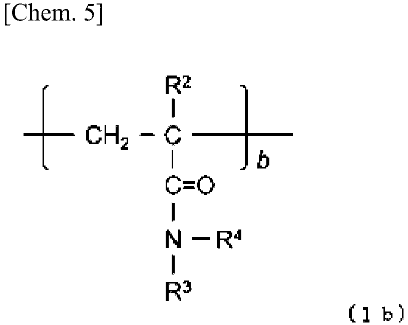

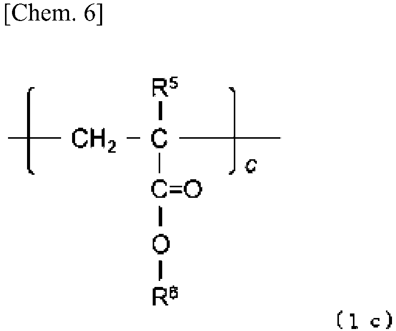

The copolymer (P) used in the ophthalmic composition of the present invention has the following three constitutional units (1) to (3), and the molar ratio of each constitutional unit of a: b: c is 100: 10 to 400: 2 to 50.

### <(1) PC Constitutional Unit>

The copolymer (P) used in the ophthalmic composition of the present invention has a constitutional unit represented by the following formula (1a) (hereinafter abbreviated as "PC constitutional unit"). The PC constitutional unit is a constitutional unit derived from a PC monomer represented by the formula (2) mentioned later. When the copolymer (P) contains the constitutional unit derived from the PC monomer, corneal cells are protected.

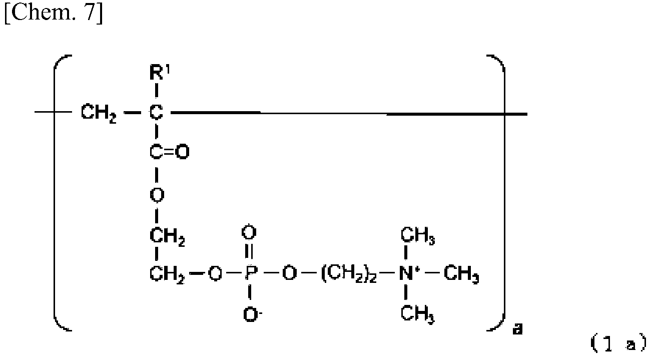

In the formula (1a), R¹ represents a hydrogen atom or a methyl group. "a" represents the ratio of the number of the constitutional unit represented by the formula (1a) in the copolymer (P), and represents the molar ratio with respect to the constitutional units represented by the formula (1b) and the constitutional unit represented by the formula (1c).

The PC constitutional unit in the copolymer (P) is introduced in order to impart hydrophilicity to the copolymer (P) and increase the effect of protecting corneal cells.

The PC constitutional unit in the copolymer (P) is obtained from a phosphorylcholine-like group-containing monomer (hereinafter, abbreviated as a PC monomer) represented by the following formula (2), which is used in polymerization of the copolymer (P). 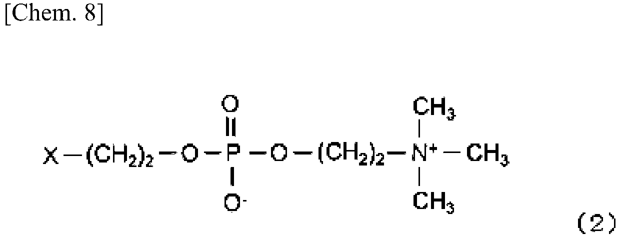 In the formula (2), X is a (meth) acryloyloxy group. Note that, the (meth) acryloyl refers to both methacryloyl and acryloyl.

As the PC monomer, from the viewpoint of availability, for example, 2-((meth) acryloyloxy) ethyl-2'-(trimethylammonio) ethyl phosphate is preferable, and 2-(methacryloyloxy) ethyl-2'-(trimethylammonio) ethyl phosphate (hereinafter referred to as MPC) represented by the following formula (3) is more preferable.

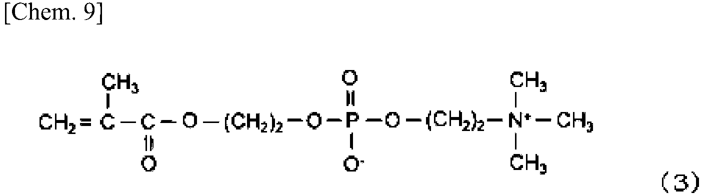

### <(2) Amide Constitutional Unit >

The copolymer (P) used in the ophthalmic composition of the present invention has a constitutional unit represented by the following formula (1b) (hereinafter, abbreviated as "amide constitutional unit").

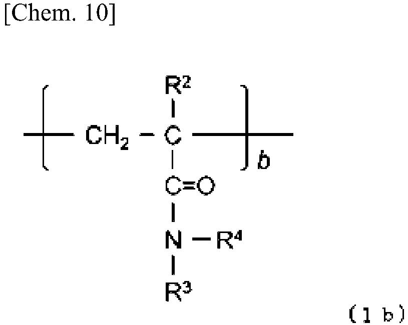

In the formula (1b), R² represents a hydrogen atom or a methyl group, and R³ and R⁴ each independently represent a hydrogen atom, a methyl group, or an ethyl group. Among these, R³ and R⁴ are each independently preferably a methyl group or an ethyl group, and more preferably a methyl group. "b" represents the ratio of the number of the constitutional unit represented by the formula (1b) in the copolymer (P), and represents the molar ratio with respect to the constitutional units represented by the formula (1a) and the constitutional unit represented by the formula (1c).

The amide constitutional unit in the copolymer (P) is introduced in order to increase the molecular weight of the copolymer (P) and increase the adhesiveness to corneal cells.

The ratio of the amide constitutional unit in the copolymer (P) is b/a = 10/100 or more and 400/100 or less, and preferably 30 / 100 or more and 250/100 or less, in the case of b where a of the PC constitutional unit is 100. When b is too large, there is a possibility that it is difficult to perform sterile filtration necessary for the production of the ophthalmic composition, and when b is too small, the effect of protecting corneal cells cannot be expected.

The amide constitutional unit in the copolymer (P) is obtained from a monomer represented by the following formula (1b') used in the polymerization of the copolymer (P), that is, (meth) acryl amide or a (meth) acryl amide derivative. In other words, the amide constitutional unit is a constitutional unit derived from a monomer represented by the formula (1b').

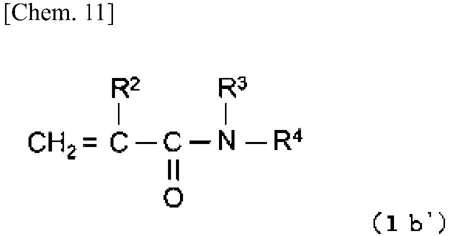

R², R³, and R⁴ in the formula (1b') are the same as R², R³, and R⁴ in the formula (1b), respectively.

Examples of the (meth) acrylamide or (meth) acrylamide derivative represented by the above formula (1b') include N,N-dimethyl (meth) acrylamide or N,N-diethyl (meth) acrylamide, and N,N -dimethylacrylamide is preferable.

### <(3) Hydrophobic Constitutional Unit>

The copolymer (P) used in the ophthalmic composition of the present invention has a constitutional unit represented by the following formula (1c) (hereinafter, abbreviated as "hydrophobic constitutional unit").

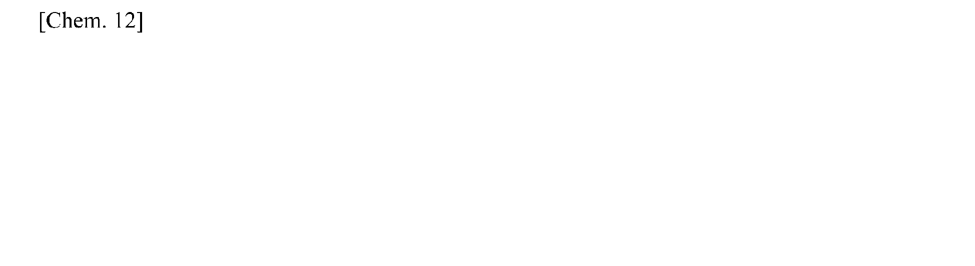

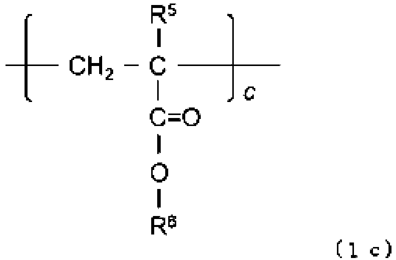

In the formula (1c), R⁵ represents a hydrogen atom or a methyl group, and R⁶ represents a monovalent hydrocarbon group having 12 to 24 carbon atoms, for example, a lauryl group, a stearyl group or a behenyl group. R⁶ is a monovalent hydrocarbon group having preferably 12 to 20 carbon atoms, more preferably 14 to 20 carbon atoms, and further more preferably 17 to 19 carbon atoms. "c" represents the ratio of the number of the constitutional unit represented by the formula (1c) in the copolymer (P), and represents the molar ratio with respect to the constitutional units represented by the formula (1a) and the formula (1b).

The hydrophobic constitutional unit in the copolymer (P) is introduced in order to increase adhesiveness to corneal cells.

The ratio of the hydrophobic constitutional unit in the copolymer (P) is c/a = 2/100 or more and 50/100 or less, and preferably 5/100 or more and 25/100 or less, in the case of c where a of the PC constitutional unit is 100. When c is too large, the hydrophilicity of the copolymer (P) is reduced, and thus its solubility in aqueous solution is reduced, and the production of the ophthalmic composition becomes difficult. When c is too small, the adhesiveness to corneal cells is reduced, and the effect of protecting corneal cells cannot be expected.

The hydrophobic constitutional unit in the copolymer (P) is obtained from a hydrophobic monomer represented by the following formula (1c') used in the polymerization of the copolymer (P). In other word, the hydrophobic constitutional unit is a constitutional unit derived from a monomer represented by the formula (1c').

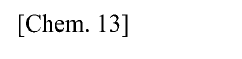

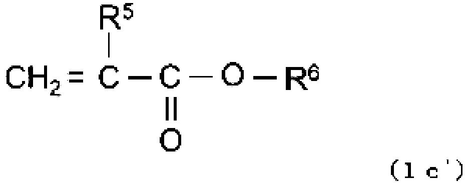

R⁵ and R⁶ in the formula (1c') are the same as R⁵ and R⁶ in the formula (1c), respectively.

Examples of the hydrophobic monomer represented by the formula (1c') include a linear alkyl (meth) acrylate such as lauryl (meth) acrylate, stearyl (meth) acrylate, and behenyl (meth) acrylate, and stearyl methacrylate is preferable.

In addition, the weight average molecular weight of the copolymer (P) is 5,000 to 2,000,000. When the weight average molecular weight is less than 5,000, there is a possibility that the effect of protecting corneal cells is not expected, and when it exceeds 2,000,000, there is a possibility that it is difficult to perform sterile filtration necessary for the production of the ophthalmic composition. Further, from the viewpoint of improving the effect of protecting corneal cells, the weight average molecular weight of the copolymer (P) is preferably 10,000 to 1,000,000, and more preferably 50,000 to 500,000. Note that the weight average molecular weight means a value in terms of polyethylene glycol (PEG) measured by gel permeation chromatography (GPC).

The copolymer (P) represented by the formula (1) has each constitutional unit derived from the formula (1a) to formula (1c), but the arrangement of each constitutional unit in the copolymer (P) is not particularly limited, and may be random or block.

The copolymer (P) of the component (A) can be produced, for example, as follows. The copolymer (P) can be produced by radical polymerization of a polymer composition containing various monomers for forming each constitutional unit of the formula (1a) to formula (1c), that is, the PC monomer, the monomer represented by the formula (1b'), and a monomer composition containing the monomer represented by the formula (1c') under the presence of a radical polymerization initiator and under substitution or atmosphere of inert gas such as nitrogen gas, carbon dioxide gas, argon gas, or helium gas. The polymerization method can be performed by a known method such as bulk polymerization, suspension polymerization, emulsion polymerization, or solution polymerization. The copolymer (P) can be purified by a known method such as a reprecipitation method, a dialysis method, or an ultrafiltration method.

Examples of the radical polymerization initiator can include an azo-based radical polymerization initiator, organic peroxide, and persulfate.

Examples of the azo-based radical polymerization initiator include 2,2-azobis (2-diaminopropyl) dihydrochloride, 2,2-azobis (2-(5-methyl-2-imidazolin-2-yl) propane) dihydrochloride, 4,4-azobis (4-cyanovaleric acid), 2,2-azobisisobutyramide dihydrate, 2,2-azobis (2,4-dimethylvaleronitrile), and 2,2-azobisisobutyronitrile (AIBN).

Examples of the organic peroxide include benzoyl peroxide, diisopropyl peroxydicarbonate, t-butylperoxy-2-ethylhexanoate, lauroyl peroxide, t-butylperoxyneodecanate, and succinic acid peroxide.

Examples of the persulfate include ammonium persulfate, potassium persulfate, and sodium persulfate.

These radical polymerization initiators can be used alone or can be used as a mixture of two or more thereof. The use amount of the polymerization initiator is usually 0.001 w/w% or more and 10 w/w% or less, and preferably 0.01 w/w% or more and 5.0 w/w% or less, with respect to 100 w/w% of the monomer composition.

The production of the copolymer (P) can be performed in the presence of a solvent. The solvent may be any solvent as long as it dissolves the monomer composition and does not react, and examples thereof can include an alcohol-based solvent, a ketone-based solvent, an ester-based solvent, a linear or cyclic ether-based solvent, and a nitrogen-containing solvent.

Examples of the alcohol-based solvent include water, methanol, ethanol, n-propanol, and isopropanol.

Examples of the ketone-based solvent include acetone, methyl ethyl ketone, and diethyl ketone.

Examples of the ester-based solvent include ethyl acetate.

Examples of the linear or cyclic ether-based solvent include ethyl cellosolve, tetrahydrofuran, and N-methylpyrrolidone.

Examples of the nitrogen-containing solvent include acetonitrile and nitromethane. Preferable examples thereof include water, alcohol, and a mixed solvent thereof.

The blending amount of the component (A) in the ophthalmic composition of the present invention is 0.01 w/w% or more and 0.5 w/w% or less. When it is less than 0.01 w/w%, there is a possibility that the effect of protecting corneal cells is not sufficiently exhibited, and when it is more than 0.5 w/w%, there is a possibility that sterile filtration necessary for the production of the ophthalmic composition is difficult. From the viewpoint of exhibiting a more sufficient effect of protecting cells, the blending amount of the component (A) is preferably 0.02 w/w% or more and 0.4 w/w% or less, more preferably 0.03 w/w% or more and 0.3 w/w% or less, and further more preferably 0.05 w/w% or more and 0.2 w/w% or less.

The ophthalmic composition of the present invention contains at least one of (B) benzalkonium chloride and (C) chlorhexidine gluconate, as a preservative. That is, the ophthalmic composition of the present invention may contain, as the preservative, (B) benzalkonium chloride (component (B)) alone, (C) chlorhexidine gluconate (component (C)) alone, or both of the component (B) and the component (C).

When the component (B) is blended in the ophthalmic composition of the present invention, the blending amount of benzalkonium chloride as the component (B) in the ophthalmic composition is 0.001 w/w% or more and 0.02 w/w% or less. When it is less than 0.001 w/w%, there is a possibility that sufficient preservative efficacy is not exhibited, and when it is more than 0.02 w/w%, there is a possibility that corneal epithelial disorder is caused. From the viewpoint of exhibiting a more sufficient effect of protecting cells, the blending amount of the component (B) is preferably 0.005 w/w% or more and 0.015 w/w% or less.

When the component (C) is blended in the ophthalmic composition of the present invention, the blending amount of chlorhexidine gluconate as the component (C) in the ophthalmic composition is 0.001 w/w% or more and 0.05 w/w% or less. When it is less than 0.001 w/w%, there is a possibility that sufficient preservative efficacy is not exhibited, and when it is more than 0.05 w/w%, there is a possibility that corneal epithelial disorder is caused. From the viewpoint of exhibiting a more sufficient effect of protecting cells, the blending amount of the component (C) is preferably 0.005 w/w% or more and 0.015 w/w% or less.

The ophthalmic composition of the present invention preferably contains 0.5 w/w% or less of hydroxypropyl methylcellulose. When it is more than 0.5 w/w%, there is a possibility that it is difficult to perform sterile filtration necessary for the production of the ophthalmic composition. From the viewpoint of sterile filtration, it is preferably 0.3 w/w% or less.

When the hydroxypropyl methylcellulose is blended in the ophthalmic composition of the present invention, the blending amount of hydroxypropyl methylcellulose in the ophthalmic composition is preferably 0.01 w/w% or more, and more preferably 0.05 w/w% or more.

The hydroxypropyl methylcellulose may be one used in an ordinary pharmaceutical product, and for example, a product listed in the Japanese Pharmacopoeia can be used.

The ophthalmic composition of the present invention may contain other components in addition to the components (A) to (C). Examples of the other components can include polyhydric alcohol, a refreshing agent, an inorganic salt, a salt of organic acid, acid, base, an antioxidant, and a mucin secretagogue.

Examples of the polyhydric alcohol include propylene glycol, glycerin, glucose, mannitol, sorbitol, xylitol, and trehalose.

Examples of the refreshing agent include menthol and camphor.

Examples of the inorganic salt include potassium chloride, sodium chloride, borax, sodium hydrogen carbonate, sodium hydrogen phosphate, and anhydrous sodium dihydrogen phosphate.

Examples of the salt of organic acid include sodium citrate and sodium acetate.

Examples of the acid include boric acid, phosphoric acid, citric acid, sulfuric acid, acetic acid, and hydrochloric acid.

Examples of the base include sodium hydroxide, potassium hydroxide, trometamol, and monoethanolamine.

Examples of the antioxidant include tocopherol acetate and dibutylhydroxytoluene. Examples of the mucin secretagogue include diquafosol sodium and rebamipide.

### Examples

Hereinafter, the present invention is specifically described with reference to examples, but the present invention is not limited to the scope of the following examples.

### [Evaluation]

Examples and Comparative Examples were evaluated as follows.

### (1) Evaluation of Cell Survival Rate

The evaluation of the cell survival rate was performed using rabbit corneal epithelial cells (SIRC cells). SIRC cells pre-cultured in advance in Dulbecco's modified eagle medium (DMEM) containing 10 v/v% FBS (fetal bovine serum) were treated with trypsin to prepare 1.0 × 10⁵ cells/mL of cell-suspension. The cell suspension was seeded in a 96-well plate at 0.1 mL/well and cultured for 24 hours to allow the cells to adhere, and then each of the ophthalmic compositions of Examples 1 to 11 and Comparative Examples 1 to 9 was added at 0.05 mL/well every 1 hour for a total of 5 times, and further cultured for 24 hours. After the culture, the medium was removed, and a 50 µg/mL of neutral red solution was added to allow the living cells to take up neutral red. After 3 hours, the cells were washed with PBS (phosphate buffer saline), neutral red was extracted with a 1 v/v% acetic acid/ 50 v/v% ethanol aqueous solution, and the absorbance of 540 nm was measured. The absorbance when the sample solution was not added was set at 100% cell survival rate. The cell survival rate to which each of the ophthalmic compositions of Examples 1 to 11 and Comparative Examples 1 to 9 was added was calculated, and the 50% inhibition concentration (IC₅₀) was calculated. When the IC₅₀ was 15% or more and less than 100%, it was determined as good "A" indicating that the cell survival rate was high, when the IC₅₀ was 10% or more and less than 15%, it was determined as fair "B" indicating that the cell survival rate was medium, and when the IC₅₀ was more than 0% and less than 10%, it was determined as poor "C" indicating that the cell survival rate was low.

### (2) Preservative Efficacy Test

The preservative efficacy test was performed in accordance with the section of "Japanese Pharmacopoeia, 18th Edition, General Information, Preservative Efficacy Test Method". Three species of Escherichia coli (E. coli, hereinafter abbreviated as E.c.) (NBRC3972), Pseudomonas aeruginosa (P. aeruginosa, hereinafter abbreviated as P.a.) (NBRC13275), and Staphylococcus aureus (S. aureus, hereinafter abbreviated as S.a.) (NBRC13276) were used as the bacterium, and two species of Candida albicans (candida, hereinafter abbreviated as C.a.) (NBRC1594) and Aspergillus brasiliensis (black aspergillus, hereinafter abbreviated as A.b.) (NBRC9455) were used as the fungus.

The BIOBALL (registered trademark) (manufactured by bioMérieux Japan Co., Ltd.) of each of E.c., P.a., S.a., C.a. and A.b. describe above was redissolved to prepare a test bacterial solution having 10⁸ CFU (Colony Forming Unit, the number of units capable of forming colonies) per 1 mL. The test bacterial solution was added to each of the ophthalmic compositions of Examples 1 to 11 and Comparative Examples 1 to 9 so as to be 1 v/v%, and the number of inoculated bacteria was adjusted to be 10⁶ CFU per 1 mL, and stored at 25°C.

For individual bacterial species, sampling was performed 14 days and 28 days after inoculation, the samples were cultured, and the number of viable bacteria was measured. A case where no colony was generated after 28 days was determined as good "A", a case where a colony was generated after 28 days but the number of bacteria after 28 days was not increased from the number of bacteria after 14 days was determined as fair "B", and a case where the number of bacteria after 28 days was increased from the number of bacteria after 14 days was determined as poor "C".

In addition, the overall evaluation was determined as good "A" in a case where a colony was not generated for all the bacterial species after 28 days, as fair "B" in a case where a colony was generated after 28 days but the number of bacteria was less than the number of bacteria after 14 days, and as poor "C" in a case where the number of bacteria after 28 days increased more than the number of bacteria after 14 days in at least one or more bacterial species.

Copolymers and homopolymers used in the following Examples and Comparative Examples are as follows.

### [Synthesis Example] (Synthesis of Copolymer P)

Into a four-necked flask, 31.8 g of 2-methacryloyloxyethyl phosphorylcholine (MPC, manufactured by NOF Corporation), 3.6 g of stearyl methacrylate (SMA, manufactured by NOF Corporation) and 9.6 g of N,N-dimethyl acrylamide (DMAA, manufactured by KJ Chemicals Corporation) were added, dissolved in 55.0 g of ethanol, and nitrogen gas was blown thereinto for 30 minutes. Then, the temperature was raised to 48°C, and 0.10 g of a polymerization initiator (PERBUTYL ND (PB-ND), manufactured by NOF Corporation) was added to perform a polymerization reaction for 8 hours. After the polymerization reaction, the polymerization liquid was added dropwise to 3 liters of diethyl ether while stirring, and the deposited precipitate was filtered and vacuum-dried at room temperature for 48 hours to obtain a powder. The yield was 40.2 g.

Copolymer (P): copolymer of MPC, DMAA, and SMA (copolymerization composition ratio of MPC, DMAA, and SMA [MPC/DMAA/SMA (molar ratio) = 10/9/1], weight average molecular weight: 100,000)
Note that the weight average molecular weight of the copolymer (P) was measured as follows.

### [Measurement of Weight Average Molecular Weight]

Five mg of the obtained copolymer (P) were dissolved in a methanol/chloroform mixture solution (80: 20) to prepare a sample solution. The concentration of the copolymer (P) in the sample solution was set to 0.5% by mass. The following analysis conditions were used.
Column: PLgel-mixed-C
Standard substance: Polyethylene glycol
Detector: Differential refractometer RI-8020 (manufactured by Tosoh Corporation)
Calculation method of weight average molecular weight: Molecular weight calculation program (GPC program for SC-8020)
Flow rate: 1 mL/min
Injection amount: 100 µL
Column oven: Constant temperature around 40°C

### [Preparation of Ophthalmic Composition]

### (Example 1)

About 50 g of purified water was heated to 80°C, and 0.1 g of hydroxypropylmethylcellulose ["60SH-50 (hypromellose)" manufactured by Shin-Etsu Chemical Co., Ltd.] was added thereto, followed by stirring. After uniform dispersion was visually confirmed, it was cooled to 47.5°C and further stirred. While maintaining the temperature at 47.5 °C, 0.62 g of sodium chloride, 0.1 g of potassium chloride, 0.4 g of boric acid, 0.04 g of borax, 0.01 g of benzalkonium chloride ["Nissan Cation M₂-100R" manufactured by NOF Corporation] as the component (B), and 0.1 g of the copolymer (P) as the component (A) were sequentially added and stirred. Then, purified water was added to be the total amount of 100 g. Thereafter, filtration sterilization was performed to obtain a sterile ophthalmic composition. The content (w/w%) of each component in the ophthalmic composition was shown in Table 1.

### (Examples 2 to 11)

Production was performed by the same procedure as in Example 1 using the components of the types and amounts shown in Table 1. Note that components and blending amounts common to Examples and Comparative Examples are shown in Table 2 as common components.

### (Comparative Examples 1 to 9)

Production was performed by the same procedure as in Example 1 using the components of the types and amounts shown in Table 3.

**[Table 1]**

| **Blending** Component (w/w%) | Example 1 | Example 2 | Example 3 | Example 4 | Example 5 | Example 6 |
|---|---|---|---|---|---|---|
| Copolymer (P) | 0.10 | 0.10 | 0.20 | 0.01 | 0.10 | 0.10 |
| Benzalkonium Chloride | 0.01 | - | 0.01 | 0.01 | 0.02 | 0.001 |
| Chlorhexidine Gluconate | - | 0.01 | - | - | - | - |
| Common Components | 1.26 | 1.26 | 1.26 | 1.26 | 1.26 | 1.26 |

**[Table 1] (continued)**

| **Blending** Component (w/w%) | Example 7 | Example 8 | Example 9 | Example 10 | Example 11 |
|---|---|---|---|---|---|
| Copolymer (P) | 0.20 | 0.01 | 0.10 | 0.10 | 0.20 |
| Benzalkonium Chloride | - | - | - | - | 0.02 |
| Chlorhexidine Gluconate | 0.01 | 0.01 | 0.05 | 0.001 | - |
| Common Components | 1.26 | 1.26 | 1.26 | 1.26 | 1.26 |

**[Table 2]**

| | Blending Component (w/w%) | |
|---|---|---|
| Common Components | Sodium Chloride | 0.62 |
| | Potassium Chloride | 0.10 |
| | Hypromellose | 0.10 |
| | Boric Acid | 0.40 |
| | Borax | 0.04 |

**[Table 3]**

| Blending Component (w/w%) | Comparative Example 1 | Comparative Example 2 | Comparative Example 3 | Comparative Example 4 | Comparative Example 5 |
|---|---|---|---|---|---|
| Copolymer (P) | - | 1.00 | 0.10 | 0.005 | 0.10 |
| Benzalkonium Chloride | 0.01 | 1.00 | - | 0.01 | 0.10 |
| Chlorhexidine Gluconate | - | - | - | - | - |
| Common Components | 1.26 | 1.26 | 1.26 | 1.26 | 1.26 |

**[Table 3] (continued)**

| Blending Component (w/w%) | Comparative Example 6 | Comparative Example 7 | Comparative Example 8 | Comparative Example 9 |
|---|---|---|---|---|
| Copolymer (P) | 0.10 | 0.10 | 0.10 | 0.005 |
| Benzalkonium Chloride | 0.0005 | - | - | 0.0005 |
| Chlorhexidine Gluconate | - | 0.10 | 0.0005 | - |
| Common Components | 1.26 | 1.26 | 1.26 | 1.26 |

The results of the cell survival rate are shown in Table 4. In Comparative Example 1 in which the copolymer (P) was not blended and Comparative Examples 4 and 9 in which the blending amount of the copolymer (P) was small, the cell survival rate was low, but in Examples 1 to 11 in which the copolymer (P) was blended, the cell survival rate was high. Accordingly, the ophthalmic composition of the present invention protects corneal cells.

### [Table 4]

**Table 4**

| | Example 1 | Example 2 | Example 3 | Example 4 | Example 5 | Example 6 |
|---|---|---|---|---|---|---|
| IC₅₀ (%) | 15 | 34 | 25 | 10 | 10 | 28 |
| Determination | A | A | A | B | B | A |

**Table 4 (continued)**

| | Example 7 | Example 8 | Example 9 | Example 10 | Example 11 |
|---|---|---|---|---|---|
| IC₅₀ (%) | 40 | 14 | 14 | 45 | 14 |
| Determination | A | B | B | A | B |

**Table 4 (continued)**

| | Comparative Example 1 | Comparative Example 2 | Comparative Example 3 | Comparative Example 4 | Comparative Example 5 |
|---|---|---|---|---|---|
| IC₅₀ (%) | 8 | 9 | 60 | 7 | 5 |
| Determination | C | C | A | C | C |

**Table 4 (continued)**

| | Comparative Example 6 | Comparative Example 7 | Comparative Example 8 | Comparative Example 9 |
|---|---|---|---|---|
| IC₅₀ (%) | 35 | 7 | 51 | 9 |
| Determination | A | C | A | C |

The results of the preservative efficacy test are shown in Table 5. Comparative Example 3 in which benzalkonium chloride was not contained and Comparative Examples 6, 8, and 9 in which the blending amount of benzalkonium chloride or chlorhexidine gluconate was small did not satisfy the determination criteria. On the other hand, Examples 1 to 11 satisfied the determination criteria even in all bacteria and fungi. Accordingly, the ophthalmic composition of the present invention exhibits sufficient preservative efficacy.

### [Table 5]

**Table 5**

| | Determination | | | | | Overall Evaluation |
|---|---|---|---|---|---|---|
| | E.c. | P.a. | S.a. | C.a. | A.b. | |
| Example 1 | A | A | A | A | A | A |
| Example 2 | A | A | A | A | A | A |
| Example 3 | A | A | A | A | A | A |
| Example 4 | A | A | A | A | A | A |
| Example 5 | A | A | A | A | A | A |
| Example 6 | B | A | B | A | A | B |
| Example 7 | A | A | A | A | A | A |
| Example 8 | A | A | A | A | A | A |
| Example 9 | A | A | A | A | A | A |
| Example 10 | B | B | A | A | A | B |
| Example 11 | A | A | A | A | A | A |

**Table 5 (continued)**

| | Determination | | | | | Overall Evaluation |
|---|---|---|---|---|---|---|
| | E.c. | P.a. | S.a. | C.a. | A.b. | |
| Comparative Example 1 | A | A | A | A | A | A |
| Comparative Example 2 | A | A | A | A | A | A |
| Comparative Example 3 | C | C | C | C | C | C |
| Comparative Example 4 | A | A | A | A | A | A |
| Comparative Example 5 | A | A | A | A | A | A |
| Comparative Example 6 | C | B | C | B | B | C |
| Comparative Example 7 | A | A | A | A | A | A |
| Comparative Example 8 | C | C | B | B | B | C |
| Comparative Example 9 | C | B | C | B | B | C |

The results of all the test items are shown in Table 6. From the results, the ophthalmic composition of the present invention exhibits sufficient preservative efficacy and protects corneal cells.

### [Table 6]

**Table 6**

| Test Items | Example 1 | Example 2 | Example 3 | Example 4 | Example 5 | Example 6 |
|---|---|---|---|---|---|---|
| Cell Survival Rate | A | A | A | B | B | A |
| Preservative Efficacy Test | A | A | A | A | A | B |

**Table 6 (continued)**

| Test Items | Example 7 | Example 8 | Example 9 | Example 10 | Example 11 |
|---|---|---|---|---|---|
| Cell Survival Rate | A | B | B | A | B |
| Preservative Efficacy Test | A | A | A | B | A |

**Table 6 (continued)**

| Test Items | Comparative Example 1 | Comparative Example 2 | Comparative Example 3 | Comparative Example 4 | Comparative Example 5 |
|---|---|---|---|---|---|
| Cell Survival Rate | C | C | A | C | C |
| Preservative Efficacy Test | A | A | C | A | A |

**Table 6 (continued)**

| Test Items | Comparative Example 6 | Comparative Example 7 | Comparative Example 8 | Comparative Example 9 |
|---|---|---|---|---|
| Cell Survival Rate | A | C | A | C |
| Preservative Efficacy Test | C | A | C | C |

### Industrial Applicability

According to the present invention, the ophthalmic composition that exhibits sufficient preservative efficacy and protects corneal cells can be obtained.

## Claims

1. An ophthalmic composition comprising (A) 0.01 w/w% or more and 0.5 w/w% or less of a copolymer having a constitutional unit represented by the following formula (1a) to formula (1c), a molar ratio of each constitutional unit of a: b: c = 100: 10 to 400: 2 to 50, and a weight average molecular weight of 5,000 to 2,000,000, and (B) 0.001 w/w% or more and 0.02 w/w% or less of benzalkonium chloride, and/or (C) 0.001 w/w% or more and 0.05 w/w% or less of chlorhexidine gluconate: 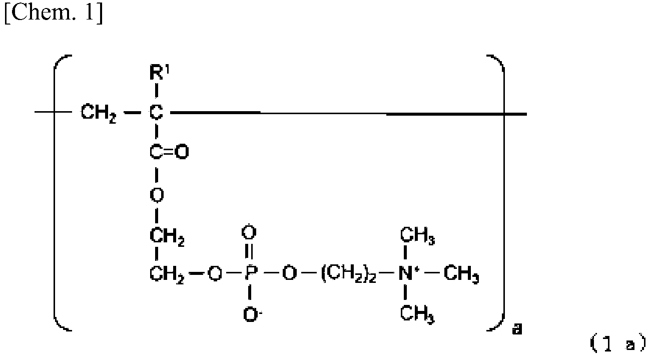 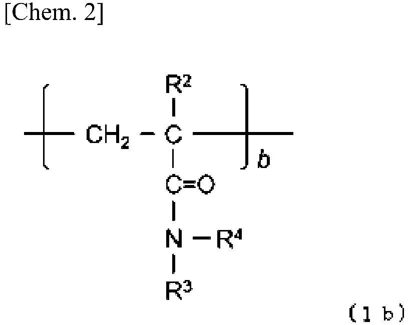 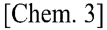 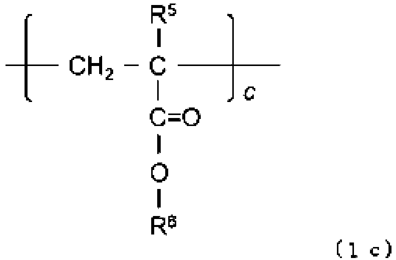 in the formula (1a) to formula (1c), a, b, and c each represent a molar ratio of each constitutional unit;
R¹, R² and R⁵ each independently represent a hydrogen atom or a methyl group;
R³ and R⁴ each independently represent a hydrogen atom, a methyl group, or an ethyl group; and
R⁶ represents a monovalent hydrocarbon group having 12 to 24 carbon atoms.

2. The ophthalmic composition according to Claim 1, comprising 0.5 w/w% or less of hydroxypropyl methylcellulose.
